# EUROPEAN PATENT APPLICATION

(11) **EP 3 138 570 A1**
(43) Date of publication of application: **08.03.2017**
(21) Application number: 15785738.4
(22) Date of filing: 24.04.2015
(51) Int. Cl.: A61K 31/7048, A61P 21/00, A61P 43/00

(54) **MUSCLE ATROPHY INHIBITOR CONTAINING QUERCETIN GLYCOSIDE**

(30) Priority: 28.04.2014 JP 2014092855
(71) Applicant: Suntory Holdings Limited, Osaka-shi, Osaka 530-8203 (JP)
(72) Inventor: OTSUKA, Yuta, Soraku-gun, Kyoto 619-0284 (JP); EGAWA, Kahori, Soraku-gun, Kyoto 619-0284 (JP); KANZAKI, Noriyuki, Soraku-gun, Kyoto 619-0284 (JP)
(74) Representative: Vossius & Partner Patentanwälte Rechtsanwälte mbB
(86) International application number: PCT/JP2015/062533
(87) International publication number: WO 2015/166887

(57) **Abstract**

An object of the present invention is to provide a new muscle atrophy inhibitor comprising a component that is safely ingestible for a long time.

It was found that glycosides of quercetin, a polyphenol present in plants, etc., have inhibitory activity on the expression of myostatin involved in muscle atrophy. This invention provides a new muscle atrophy inhibitor comprising a quercetin glycoside which is excellent in absorbability into the body and high in safety.

## Description

### TECHNICAL FIELD

The present invention relates to a muscle atrophy inhibitor comprising a quercetin glycoside.

### BACKGROUND ART

Japan has become a super-aging society, and many suggestions have been offered on various themes among middle-aged and elderly persons, such as their social participation and how to spend their leisure time. In the meantime, as the population aged, the issue of motor disorders has become obvious -- this newly emerging problem is difficult to address merely by an extension of conventional ways of thinking, because there are a huge number of cases suffering from such disorders, including severe cases and concurrent cases with two or more disorders. In particular, impairment of motor functions by muscle atrophy is regarded as one of major causes of musculoskeletal ambulation disability symptom complex, locomotive syndrome, and other disorders, since this impairment results in a vicious circle which leads to a rise in risk for falling, bone fracture, prolonged bed rest, and further muscle atrophy and further impairment of motor functions. Further, motor dysfunctions caused by muscle atrophy worsen not only quality of life (QOL) but also the prognosis of an underlying disease due to, for example, an increased frequency of concomitant metabolic disorders or infections; thus, motor dysfunctions are an urgent issue that should be solved in the face of a super-aging society. Recently, not only exercise therapies such as rehabilitation, but also nutritional approaches are studied as means for preventing the aforementioned impairment of motor functions, and components capable of boosting muscle mass have been discovered (refer to Patent Literatures 1 and 2).

Muscle atrophy is known to be caused by a variety of factors, and is believed to be particularly affected by an increase in glucocorticoid level. Dexamethasone, a type of synthetic glucocorticoid, is capable of increasing the expression of the muscle degradation-related genes Atrogin-1 and MuRF-1 in muscle tissues; so, cultured myotube cells treated with glucocorticoid are widely used as muscle atrophy models. It is known that in muscle tissues, dexamethasone binds to a glucocorticoid receptor to upregulate the expression of genes containing glucocorticoid receptor-binding sites, and then the expression of these genes takes part in enhancement of degradation of muscle proteins and in suppression of differentiation of muscle progenitor cells, *i.e.,* satellite cells (refer to Non-Patent Literature 1). The metabolic changes induced by dexamethasone resemble those changes observed during muscle atrophy in animals or humans; thus, dexamethasone-induced muscle atrophy models are widely used in the analysis of the mechanisms of muscle atrophy.

In this connection, myostatin (hereinafter abbreviated as "Mstn"), a member of the transforming growth factor-β (TGF-β) family, is known to play a part in negatively controlling muscle growth (refer to Non-Patent Literature 2). It is reported that Mstn is capable of suppressing differentiation of satellite cells, stimulating muscle degradation through the inhibition of the Akt pathway and Foxo1, and suppressing muscle synthesis via the mTOR pathway (refer to Non-Patent Literature 3). Also, it is known that no dexamethasone-induced muscle atrophy is elicited in Mstn-deficient mice (refer to Non-Patent Literature 4). Mstn affects not only an increase in muscle mass, but also enlargement of adipose tissue and deterioration of cardiac function. It is reported that in Mstn-deficient mice, as compared to wild-type mice, the size of adipose tissue mass is smaller, so that the strain on cardiac function by stress loading is reduced (refer to Non-Patent Literature 5). Hitherto, there has been a report of a Mstn inhibitor composition comprising a black tea extract (refer to Patent Literature 3).

Quercetin, a polyphenol abundant in plants, is contained as it is or in the form of a glycoside (e.g., rutin, quercitrin) in various plants including citrus fruits, onion, buckwheat, and sophora. It is known that quercetin and its glycosides have a wide variety of physiological functions, such as platelet aggregation/adhesion inhibition activities, vasodilatory activity, and anticancer activity.

### CITATION LIST

### PATENT LITERATURES

Patent Literature 1: Japanese Patent Application Publication No. JP 2009-62346
Patent Literature 2: International Patent Publication No. JP WO 2011/108487 A1
Patent Literature 3: Japanese Patent Application Publication No. JP 2013-91608

### NON-PATENT LITERATURES

Non-Patent Literature 1: Yanjun, et al., PLos One, 2013, 8(13), e58554
Non-Patent Literature 2: David, et al., Med Sci Sports Exerc, 2011, 43(10), 1828-1835
Non-Patent Literature 3: Elkina, et al., J Cachexia Sarcopenia Muscle, 2011, 2, 143-151
Non-Patent Literature 4: H. Gilson, et al., Endocrinology, 2007, 148(1), 452-460
Non-Patent Literature 5: Mellisa, et al., Journal of Endocrinology, 2012, 213, 263-275

### SUMMARY OF INVENTION

### TECHNICAL PROBLEM

In order to develop a muscle atrophy inhibitor, it is necessary to discover an inhibitory component for Mstn which is involved in stimulation of muscle degradation and suppression of muscle synthesis. However, there has hitherto been only a report that a black tea extract has Mstn inhibitory activity (refer to Patent Literature 3), and there is no observation of Mstn inhibitory activity in a single component derived from a natural product. The present invention has as its object to provide a new muscle atrophy inhibitor comprising a component that is safely ingestible for a long time.

### SOLUTION TO PROBLEM

In order to achieve the aforementioned object, the present inventors have made intensive studies in animal models of muscle atrophy induced by the synthetic glucocorticoid dexamethasone, and as a result, found that glycosides of quercetin, a polyphenol present in plants, etc., have muscle atrophy inhibitory activity. As a result of gene expression analysis, the inventors found that quercetin glycosides are capable of inhibiting the expression of Mstn which is involved in stimulation of muscle degradation and suppression of muscle synthesis. Further, the inventors found that quercetin glycosides act on various factors, such as muscle degradation pathway and muscle synthesis pathway, through the inhibition of Mstn expression, and are thereby capable of inhibiting muscle atrophy. Thus, the inventors have completed the present invention.

More specifically, the present invention is directed to the following.
1). A muscle atrophy inhibitor comprising a quercetin glycoside.
2). The muscle atrophy inhibitor as set forth in 1), wherein inhibition of muscle atrophy is triggered by inhibition of Mstn expression.
3). The muscle atrophy inhibitor as set forth in 1) or 2), wherein inhibition of muscle atrophy is triggered by inhibition of the expression of at least one gene selected from the group consisting of Atrogin-1, MuRF-1, Foxo1 and Redd1.
4). The muscle atrophy inhibitor as set forth in any of 1) to 3), wherein the muscle atrophy inhibitor has muscle degradation inhibitory activity.
5). The muscle atrophy inhibitor as set forth in 4), wherein the muscle degradation inhibitory activity is triggered by inhibition of the expression of at least one gene selected from the group consisting of Atrogin-1, MuRF-1 and Foxo1.
6). The muscle atrophy inhibitor as set forth in any of 1) to 3), wherein the muscle atrophy inhibitor has muscle synthesis stimulatory activity.
7). The muscle atrophy inhibitor as set forth in 6), wherein the muscle synthesis stimulatory activity is triggered by inhibition of the expression of Redd1.
8). The muscle atrophy inhibitor as set forth in any of 1) to 7), wherein the muscle atrophy inhibitor is intended for use in the prevention or treatment of impaired motor functions or motor disorders.
9). The muscle atrophy inhibitor as set forth in any of 1) to 7), wherein the muscle atrophy inhibitor is intended for use in the prevention or treatment of drug-induced muscle atrophy.
10). A composition comprising the muscle atrophy inhibitor as set forth in any of 1) to 9).

### ADVANTAGEOUS EFFECTS OF INVENTION

The present invention makes it possible to use a quercetin glycoside in an agent intended for inhibition of muscle atrophy. Inhibition of muscle atrophy achieved according to this invention leads to providing a new means that contributes the improvement of the quality of life of affected persons and elderly persons.

Quercetin glycosides are members of polyphenolic compounds and have various physiological activities including blood flow improving activity and anticancer activity. Moreover, those glycosides are derived from plants and so are very high in safety. Therefore, the present invention can expect not only muscle atrophy inhibitory activity but also other beneficial physiological activities of quercetin glycosides, and can provide a safe and continuously ingestible agent.

### BRIEF DESCRIPTIONS OF DRAWINGS

FIG. 1 shows the inhibitory activity of a quercetin glycoside (QG) on dexamethasone (DEX)-induced muscle atrophy.
FIG. 2 shows the inhibitory activity of a quercetin glycoside (QG) on the expression of Mstn and its downstream genes.
FIG. 3 shows a comparison between a quercetin glycoside (QG) and quercetin (Q) in terms of inhibitory activity on dexamethasone (DEX)-induced muscle atrophy.
FIG. 4 shows a comparison between a quercetin glycoside (QG) and quercetin (Q) in terms of inhibitory activity on the expression of Mstn downstream genes.

### DESCRIPTION OF EMBODIMENTS

The present invention is directed to a muscle atrophy inhibitor comprising a quercetin glycoside.

The muscle atrophy inhibitor comprising a quercetin glycoside according to this invention refers to a muscle atrophy inhibitor comprising a quercetin glycoside as an active component.

As referred to in the present invention, the "quercetin glycoside" refers to a glycoside of quercetin which is a type of polyphenol, and is represented by the following formula: 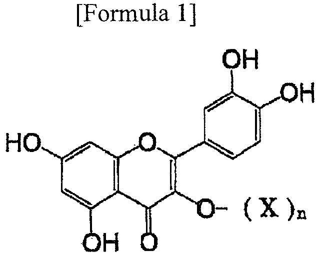 (wherein (X)ₙ represents a sugar chain, and n is an integer of 1 or more.)

In this formula, a sugar represented by X, which constitutes a sugar chain and is glycosidically bound to quercetin, is exemplified by glucose, rhamnose, galactose, and glucuronic acid, with glucose or rhamnose being preferred. Also, n is not particularly limited as long as it is an integer of 1 or more; preferably, n is an integer of 1 to 16, and more preferably n is an integer of 1 to 8. When n is 2 or more, the moiety X may be composed of one type of sugar chain or may be composed of two or more types of sugar chains.

The quercetin glycoside according to the present invention even includes those glycosides obtained by transglycosylation of existing quercetin glycosides by treatment with an enzyme or the like. The quercetin glycoside as referred to in this invention specifically includes rutin, enzyme-treated rutin, quercitrin, and isoquercitrin.

In the present invention, one compound as encompassed by the quercetin glycoside may be used alone, or two or more such compounds may be used in combination. The source and production method of the quercetin glycoside used in this invention are not particularly limited. For example, buckwheat, sophora, caper, apple, tea, onion, grape, broccoli, Jew's mallow, raspberry, lingonberry/cowberry, cranberry, Opuntia, leaf vegetables, citrus fruits and the like are known as plants rich in quercetin or quercetin glycosides, and the inventive quercetin glycoside can be obtained from such plants. Examples of the quercetin glycoside that can be used in this invention include products obtained by performing condensation, purification or any other treatment of a natural product extract to enhance a quercetin glycoside concentration -- for example, condensed or purified products of quercetin glycoside-containing extracts. Condensation or purification of the extract can be performed using an existing method.

In a particularly preferred mode of the present invention, an enzyme-treated product of rutin is used as the quercetin glycoside. Particularly preferred examples of enzyme-treated rutin include materials comprising, as a principal component, isoquercitrin which is obtained by treating a quercetin glycoside with an enzyme to remove a rhamnose sugar chain moiety, a transglycosylated product of isoquercitrin which is obtained by treating isoquercitrin with a glycosyltransferase to attach a sugar chain consisting of one to seven glucoses to the backbone, or a mixture thereof.

The quercetin glycoside refers to a compound in which a sugar chain is glycosidically bound to quercetin, or more specifically, is a generic name for a series of compounds in which one or more sugar chains are glycosidically bound to the hydroxy group at position 3. Quercetin and quercetin glycosides are greatly different in both chemical structure and chemical properties.

As referred to herein, the "glucocorticoid" refers to a type of adrenocortical hormone that induces ubiquitin-proteasome pathway-dependent proteolysis in skeletal muscle, and is an important factor in muscle degradation. Dexamethasone, a type of synthetic glucocorticoid, is found to be capable of enhancing the expression of the muscle degradation-related genes Atrogin-1 and MuRF1 in skeletal muscle (refer to Non-Patent Literature 1). Since the metabolic changes induced by dexamethasone resemble those changes observed during muscle atrophy in animals or humans, dexamethasone-induced muscle atrophy models are widely used in the analysis of the mechanisms of muscle atrophy.

The following provides descriptions about the genes and proteins mentioned herein.

As referred to herein, the "Akt pathway" is a generic name for signaling pathways mediated by Akt, a kinase involved in the control of various cellular functions. The Akt activity is regulated by different stimuli such as nutrition, growth factors, and exercise. Activated Akt activates S6K (ribosomal protein S6 kinase), a kinase involved in muscle synthesis, via mTOR (mammalian target of rapamycin). It is also known that activated Akt indirectly suppresses muscle degradation by inhibiting Foxo1, a factor that stimulates muscle degradation.

As referred to herein, "Mstn (myostatin)" is a protein that belongs to the TGF-β superfamily, and is specifically expressed as a myogenesis inhibitory factor in skeletal muscle, cardiac muscle and adipose tissue. Mstn is intracellularly converted into an activator, and stimulates muscle degradation and suppresses muscle synthesis by inhibiting Akt through the phosphorylation/activation of Smad (small mothers against decapentaplegic). It is reported that Mstn is capable of suppressing differentiation of satellite cells, stimulating muscle degradation through the inhibition of the Akt pathway, and suppressing muscle synthesis via the mTOR pathway (refer to Non-Patent Literature 3). Also, it is known that no dexamethasone-induced muscle atrophy is elicited in Mstn-deficient mice (refer to Non-Patent Literature 4).

As referred to herein, "Atrogin-1" and "MuRF1 (muscle RING-finger protein-1)" are both ubiquitin ligases involved in the ubiquitin-proteasome system which is one of muscle degradation pathways, and are known to be expressed in skeletal muscle and cardiac muscle.

As referred to herein, "Foxo1" refers to a forkhead transcription factor called "Forkhead box protein O1". Foxo1 is commonly located in cytoplasm in a phosphorylated state but, when dephosphorylated, it travels into the nucleus and functions as a transcription factor. Increased Foxo1 expression is common in muscle atrophy, and is known to be involved in various mechanisms such as proteolysis by the ubiquitin-proteasome system, enhancement of autophagy, and suppression of protein synthesis.

As referred to herein, "Redd1" is a protein called "regulated in development and DNA damage response 1". It is known that Redd1 is induced to be expressed under reduced oxygen conditions, etc. and suppresses muscle synthesis by inhibiting the mTOR pathway.

As referred to herein, "muscle atrophy" refers to a condition where the metabolic turnover of muscle synthesis and degradation is skewed towards more degradation so that myocytes decrease in number or diminish in size, resulting in loss of muscle mass. Muscle atrophy is broadly classified into different types caused by prolonged bed rest, cast immobilization due to bone fracture, etc., illness, and aging. Therefore, "inhibition of muscle atrophy" refers to inhibition of impairment of motor functions or loss of muscle mass due to the aforementioned causes.

As referred to herein, "muscle degradation" refers to enhancement of the degradation/catabolism of myofibrillar proteins through the induction of the expression of genes involved in the Akt pathway, cathepsin system, ubiquitin-proteasome system, autophagy system, etc. More specifically, muscle degradation refers to enhancement of myofibrillar proteolysis through the induction of the expression of Mstn genes and other genes (*e.g.,* Atrogin-1, MuRF1 and Foxo1) involved in the ubiquitin-proteasome system, a muscle proteolysis pathway.

As referred to herein, "muscle synthesis" refers to enhancement of the synthesis/anabolism of myofibrillar proteins. More specifically, muscle synthesis refers to stimulation of muscle protein synthesis through the induction of the activation of the kinase complex mTOR, a translational control factor present in skeletal muscle, by inhibition of the expression of Mstn and Redd1 genes.

The quercetin glycoside according to the present invention is capable of inhibiting the expression of factors (Mstn, Atrogin-1, MuRF-1, Foxo1 and Redd1) related to muscle metabolisms such as muscle synthesis and degradation. To be specific, as described below in Example 2, the inventive quercetin glycoside has inhibitory activity on the expression of Mstn, a skeletal myogenesis inhibitory factor (Example 2). Also, the inventive quercetin glycoside is capable of inhibiting the expression of three muscle degradation-related factors Atrogin-1, MuRF-1 and Foxo1, to suppress muscle degradation, through inhibition of the Akt pathway due to inhibition of Mstn expression (Example 2). Further, due to inhibition of Mstn expression by the inventive quercetin glycoside, the expression of Redd1 capable of inhibiting the mTOR pathway involved in muscle synthesis is inhibited to stimulate muscle synthesis (Examples 1 and 2). That is, this invention makes it possible to, for example, stimulate muscle enlargement/regeneration, improve muscle strength, regulate/increase muscle mass, and prevent or suppress muscle atrophy, by inhibiting the expression of Mstn which triggers signaling pathways involved in a series of muscular metabolism processes, *i.e.,* muscle synthesis and degradation.

Furthermore, in muscle atrophy models, the muscle atrophy inhibitory activity and the inhibitory activity on the expression of muscle atrophy-related genes were exhibited by quercetin glycosides, but not by quercetin (Example 3). Therefore, quercetin glycosides can produce superior effects which cannot be achieved by quercetin.

The muscle atrophy inhibitor of the present invention is intended for use to prevent or treat impaired motor functions or motor disorders. For example, the inventive muscle atrophy inhibitor can be applied for various uses, including but not limited to, prevention or treatment of motor disorders, locomotive syndrome and the like associated with prolonged bed rest, cast immobilization due to bone fracture, etc., illness, and aging. Such uses include uses in humans or non-human animals, and can be uses for therapeutic or non-therapeutic purposes. As referred to herein, the term "non-therapeutic" is a concept that does not include medical practice, or in other words, acts of treatment of the human body by therapy.

The muscle atrophy inhibitor of the present invention is also intended for use to prevent or treat drug-induced muscle atrophy. For example, the inventive muscle atrophy inhibitor can be applied for various uses, including but not limited to, prevention or treatment of muscle atrophy associated with prolonged ingestion of steroid hormones. Such uses include uses in humans or non-human animals, and can be uses for therapeutic or non-therapeutic purposes.

The present invention can provide the muscle atrophy inhibitor in the form of, for example, an agent such as pharmaceutical product, but the aforementioned form is not the sole example.

The muscle atrophy inhibitor comprising a quercetin glycoside according to the present invention can also be provided in the form of a composition comprising said inhibitor. For example, the inventive muscle atrophy inhibitor can be provided in the form of a pharmaceutical composition, but the aforementioned form is not the sole example.

Further, the present invention can also provide the muscle atrophy inhibitor in the form of a pet food or animal feed processed for feeding to companion animals, or a medicine for animals.

The muscle atrophy inhibitor (pharmaceutical composition, and the like) of the present invention can comprise a quercetin glycoside in an amount of about 0.1 mg to 8000 mg, preferably about 0.3 mg to 4000 mg, in terms of quercetin equivalent, though this amount varies with the total amount of the inhibitor. It is advisable that the total proportion of the quercetin glycoside to be included in the inventive inhibitor be in the range of preferably about 0.001 to 95% by weight, more preferably about 0.01 to 80% by weight, based on the total weight of the inhibitor.

In the case of administering the inventive inhibitor to an animal (in an amount of about 20 g per mouse), it is advisable that the quercetin glycoside be included in a total amount sufficient to ingest about 0.1 mg to 16 mg, preferably about 0.3 mg to 4 mg, of quercetin. In particular, in the case of administering the inventive inhibitor to a (adult) human, it is advisable that the quercetin glycoside be included in a total amount sufficient to ingest about 0.1 mg to 8000 mg, preferably about 0.3 mg to 4000 mg, of quercetin.

The amount of a quercetin glycoside to be included in the composition of the present invention can be determined using as a guide an enzyme-treated rutin intake of 0.1 to 20 g, preferably 0.3 to 10 g, per individual per day. The aforementioned amount can also be determined to give an intake of, for example, 0.002 to 400 mg/kg, more preferably 0.006 to 200 mg/kg, per kg body weight. Alternatively, the aforementioned amount can be determined to be in the range of 0.001 to 95% by weight, preferably 0.01 to 80% by weight, based on the total weight of the composition.

In the case of using the inventive muscle atrophy inhibitor as a medicament or the like, the inhibitor can be administered in an oral dosage form or in any other dosage forms such as injection -- a known formulation suitable for each administration can be used as appropriate. Examples of formulations suitable for oral administration include, but are not limited to, tablet, capsule, powder, granule, solution, suspension, and syrup.

The inventive inhibitor can, depending on its form, contain any other given additives and/or any other given components used in common agents, besides the quercetin glycoside. Examples of such additives and/or components include vitamins such as vitamin E and vitamin C, minerals, nutritional components, and physiologically active components such as flavorants, as well as other components added for making pharmaceutical preparations, such as excipients, binders, emulsifiers, isotonizers, buffers, solubilizers, antiseptics, stabilizers, antioxidants, colorants, coagulators, and coating agents.

### EXAMPLES

Hereunder, the present invention will be more specifically described by way of working examples, but these examples are not intended to limit the scope of this invention. Those skilled in the art could use this invention with various alternations or modifications being made thereto, and such alternations and modifications are also included in the scope of this invention.

### Example 1: Inhibitory activity of quercetin glycoside on dexamethasone-induced muscle atrophy

BALB/c male mice (aged 7 weeks) were purchased from Shimizu Laboratory Supplies Co., Ltd., habituated to test environment for one week, and then measured for weight on the date of completion of the habituation period. Those animals which were well grown after completion of this period were used in the test. CE-2 (solid) produced by Clea Japan, Inc. was used as a diet, and the mice were allowed to eat this diet *ad libitum* throughout the test period. The mice were also allowed to take tap water *ad libitum* for the habituation period. Four or five mice were housed per cage, and the cages were replaced twice a week.

After the mice were administered 4.5 g/L quercetin glycoside and tap water (control) in drinking water for seven days from the completion of the habituation period, the mice were subjected to a drinking water administration test of combined dexamethasone (DEX) and quercetin glycoside (QG) for seven days. DEX was dissolved in tap water at a concentration of 10 mg/L, and QG was dissolved in tap water at different concentrations of 1.5 g/L and 4.5 g/L. The test mice were divided into four groups: control (tap water) group, DEX group, DEX + 1.5 g/L QG group, and DEX + 4.5 g/L QG group. After completion of the administration test, the mice were euthanized via cervical dislocation, gastrocnemius tissue was dissected from the mice and measured for weight, and muscle atrophy evaluation was performed using a quotient of gastrocnemius weight (GW) divided by body weight (BW). The values obtained were expressed as mean ± standard deviation. The difference in average GW/BW ratio between the control group and the DEX group was analyzed by Student's t-test. The differences in average GW/BW ratio between the DEX group and the DEX + 1.5 g/L QG group or the DEX + 4.5 g/L QG group were analyzed by Dunnett's multiple comparison test (Dunnett's test).

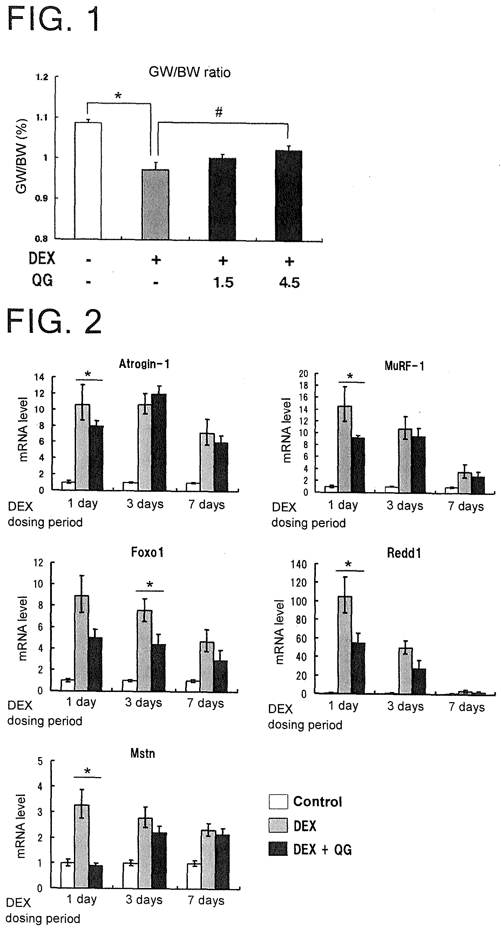

The results are shown in FIG. 1. As a result of this test, the GW/BW ratio was significantly lowered by administration of DEX in drinking water as compared to the control group (FIG. 1). In contrast, administration of combined DEX and QG in drinking water produced a significant increase in GW/BW ratio in a QG concentration-dependent manner, as compared to the DEX group (FIG. 1). The above results demonstrate that the quercetin glycoside exhibits inhibitory activity on dexamethasone-induced muscle atrophy.

### Example 2: Inhibitory activity of quercetin glycoside on the expression of Mstn and its downstream genes

BALB/c male mice aged 7 weeks were habituated for one week, and then allowed to take 4.5g/L quercetin glycoside and tap water (control) *ad libitum* for one week. After one week, the mice were allowed to take a mixture of 10 mg/L dexamethasone (DEX) and 4.5 g/L quercetin glycoside (QG) *ad libitum,* and dissected to collect left and right gastrocnemius samples on days 1, 3 and 7 after feeding. The gastrocnemius samples were instantly cooled in liquid nitrogen and refrigerated at -80°C until analysis.

Thereafter, for the purpose of a detailed analysis of the molecular mechanisms, the expression of genes involved in muscle atrophy in gastrocnemius tissue was analyzed. RNA was extracted from the cryopreserved gastrocnemius samples using ISOGEN (Nippon Gene Co., Ltd.) and the RNeazy Mini Kit (Qiagen). Then, cDNA synthesis was conducted using the High-Capacity cDNA Reverse Transcriptional Kits (Applied biosystems). Based on the constructed cDNAs, the messenger RNA (mRNA) levels of the muscle atrophy-related genes (Atrogin-1, MuRF-1, Foxo1, Redd1, and Mstn) and the control gene (18SrRNA) were determined by quantitative reverse transcription PCR using the TaqMan Fast Universal PCR Master Mix (Applied biosystems). The values obtained were expressed as mean ± standard deviation. The differences in average gene expression levels between the DEX group and each of the DEX + QG groups were analyzed by Student's t-test, with p-values below 5% being considered significant.

The results are shown in FIG. 2. As a result of the gene expression analysis, in the drinking water administration test of combined DEX and QG for one day, the DEX + QG groups showed a decreasing trend in the mRNA level of Foxo1, and a significant decrease in the mRNA levels of Atrogin-1, MuRF-1 and Redd1, as compared to the DEX group (FIG. 2). Also, the mRNA level of Mstn was completely lowered to that level in the control group by administration of combined DEX and QG in drinking water (FIG. 2).

This test demonstrated that the quercetin glycoside is capable of inhibiting the expression of Mstn, and that the quercetin glycoside is also capable of inhibiting muscle atrophy by acting on the genes downstream of Mstn which are involved in muscle differentiation, synthesis and degradation.

### Example 3: Comparison between quercetin glycoside and quercetin in terms of inhibitory activity on dexamethasone-induced muscle atrophy

BALB/c male mice aged 7 weeks were habituated for one week, and then allowed to ingest 10 mg/L dexamethasone (DEX) in drinking water *ad libitum.* Next, 200 mg/kg quercetin glycoside (QG) and an equivalent amount of quercetin (Q) in terms of rutin were each suspended in milliQ water containing 0.5% carboxymethyl cellulose sodium salt, and the suspensions were each forcibly administered orally in drinking water to the mice for five days (from Monday to Friday). Then, administration of 10 mg/L DEX in drinking water was started from Friday. In the next week, forced oral QG/Q administration was resumed (from Monday to Thursday) concurrently with DEX administration in drinking water, and the mice were dissected on Friday. Sample collection after the administration test, muscle atrophy evaluation based on gastrocnemius weight (GW)/body weight (BW) ratio, and gene expression level analysis in gastrocnemius tissue were performed according to the procedures described in Examples 1 and 2. The differences in average gene expression levels between the DEX group and each of the DEX + QG group and the DEX + Q group were analyzed by Dunnett's test, with p-values below 5% being considered significant.

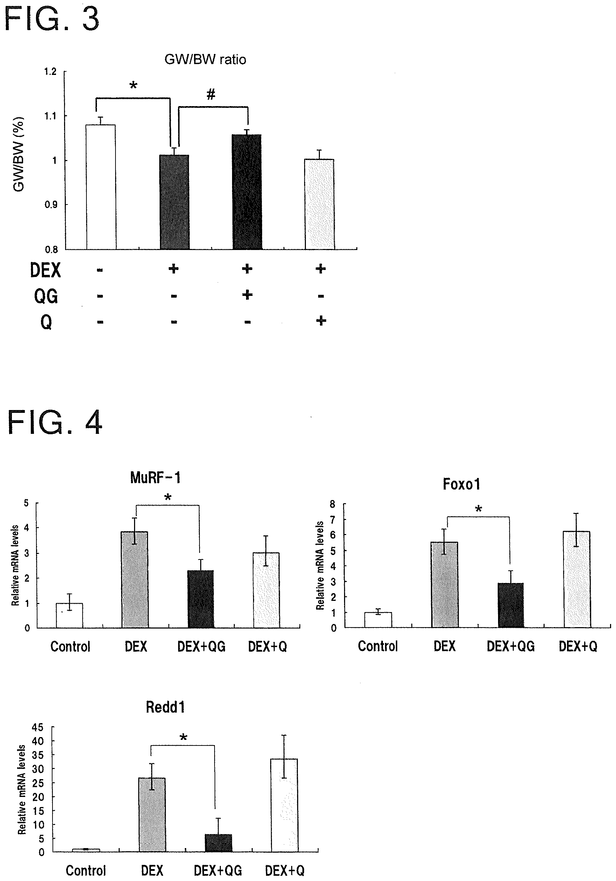

The results are shown in FIGs. 3 and 4. As a result of the forced oral administration test, the GW/BW ratio was significantly lowered by DEX administration as compared to the control group but was significantly increased by forced oral QG administration (FIG. 3). In contrast, forced oral administration of quercetin was not effective in improving the GW/BW ratio decreased by DEX administration (FIG. 3). As a result of the gene expression analysis, the expression of the muscle atrophy-related genes MuRF-1, Foxo1 and Redd1 increased by DEX administration in drinking water was significantly lowered by forced oral QG administration (FIG. 4). In contrast, forced oral administration of quercetin was not effective in inhibiting the expression of the muscle atrophy-related genes increased by DEX administration (FIG. 4). The above results demonstrate that the quercetin glycoside exhibits muscle atrophy inhibitory activity which is not exhibited by quercetin.

### INDUSTRIAL APPLICABILITY

The muscle atrophy inhibitor of the present invention comprises a quercetin glycoside which is excellent in absorbability into the body, and thus can exhibit expected physiological activities at low doses or intakes. Also, since quercetin glycosides are plant-derived components, they are excellent in safety and less likely to develop an unexpected adverse event associated with the taking or ingestion. Therefore, the inventive muscle atrophy inhibitor comprising a quercetin glycoside can achieve a superior muscle atrophy inhibitory effect safely at low doses, and thus is high in industrial applicability as a new means for preventing and treating motor disorders caused by muscle atrophy and the like.

## Claims

1. A muscle atrophy inhibitor comprising a quercetin glycoside.

2. The muscle atrophy inhibitor according to claim 1, wherein inhibition of muscle atrophy is triggered by inhibition of Mstn expression.

3. The muscle atrophy inhibitor according to claim 1 or 2, wherein inhibition of muscle atrophy is triggered by inhibition of the expression of at least one gene selected from the group consisting of Atrogin-1, MuRF-1, Foxo1 and Redd1.

4. The muscle atrophy inhibitor according to any one of claims 1 to 3, wherein the muscle atrophy inhibitor has muscle degradation inhibitory activity.

5. The muscle atrophy inhibitor according to claim 4, wherein the muscle degradation inhibitory activity is triggered by inhibition of the expression of at least one gene selected from the group consisting of Atrogin-1, MuRF-1 and Foxo1.

6. The muscle atrophy inhibitor according to any one of claims 1 to 3, wherein the muscle atrophy inhibitor has muscle synthesis stimulatory activity.

7. The muscle atrophy inhibitor according to claim 6, wherein the muscle synthesis stimulatory activity is triggered by inhibition of the expression of Redd1.

8. The muscle atrophy inhibitor according to any one of claims 1 to 7, wherein the muscle atrophy inhibitor is intended for use in the prevention or treatment of impaired motor functions or motor disorders.

9. The muscle atrophy inhibitor according to any one of claims 1 to 7, wherein the muscle atrophy inhibitor is intended for use in the prevention or treatment of drug-induced muscle atrophy.

10. A composition comprising the muscle atrophy inhibitor according to any one of claims 1 to 9.
